Europäisches Patentamt

European Patent Office

Office européen des brevets

(11) Veröffentlichungsnummer : **0 182 040**
**B1**

(12) **EUROPÄISCHE PATENTSCHRIFT**

(45) Veröffentlichungstag der Patentschrift :
**30.11.88**

(51) Int. Cl.⁴ : **A 61 B   6/03**, A 61 B   6/00

(21) Anmeldenummer : **85112046.9**

(22) Anmeldetag : **24.09.85**

(54) **Kühlvorrichtung für einen Computertomographen.**

(30) Priorität : **08.10.84 DE 3436867**

(43) Veröffentlichungstag der Anmeldung :
**28.05.86 Patentblatt 86/22**

(45) Bekanntmachung des Hinweises auf die Patenterteilung : **30.11.88 Patentblatt 88/48**

(84) Benannte Vertragsstaaten :
**DE FR**

(56) Entgegenhaltungen :
**EP-A- 0 093 468**
**EP-A- 0 109 206**
**GB-A- 1 527 813**
**GB-A- 2 026 812**

(73) Patentinhaber : **Siemens Aktiengesellschaft Berlin und München**
**Wittelsbacherplatz 2**
**D-8000 München 2 (DE)**

(72) Erfinder : **Hahn, Günter**
**Johannisstrasse 8**
**D-8500 Nürnberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 182 040 B1

Jouve, 18, rue St-Denis, 75001 Paris, France

## Beschreibung

Die Erfindung betrifft eine Computertomographen mit einem feststehenden Geräteteil, in dem ein Drehkranz drehbar gelagert ist, der zu beiden Seiten einer den Patienten aufnehmenden Öffnung einen Röntgenstrahler und einen Strahlendetektor trägt.

Bei einem Computertomographen dieser Art muß die Verlustwärme der Röntgenröhre und gegebenenfalls auch des Strahlendetektors und/oder der Meßelektronik abgeführt werden. Hierzu kann ein Ölkreislauf vorgesehen werden, in dem Kühlöl zwischen der zu kühlenden Komponente und einem durch ein Gebläse gekühlten Ölkühler zirkuliert. Der Ölkühler muß dabei auf dem Drehkranz angeordnet werden, wodurch eine starke Erwärmung des Innenraumes des Computertomographen bedingt ist. Außerdem können sich Wärmenester ausbilden. Dadurch wird in starkem Maße die empfindliche Meßelektronik negativ beeinflußt.

Der Erfindung liegt die Aufgabe zugrunde, einen Computertomographen der eingangs genannten Art so auszubilden, daß eine gezielte Abführung der Verlustwärme vom Drehkranz nach außen erfolgt.

Diese Aufgabe ist erfindungsgemäß dadurch gelöst, daß die dem Drehkranz zugewandte Fläche des feststehenden Geräteteiles einen U-förmigen Querschnitt aufweist und mit der dem feststehenden Geräteteil zugewandten Fläche des Drehkranzes einen Ringkanal bildet, der mindestens eine Einströmöffnung und eine Ausströmöffnung für ein Kühlmedium aufweist und in den am Drehkranz angeordnete Kühlrippen ragen. Bei dem erfindungsgemäßen Computertomographen können am Drehkranz längs eines Kreises Kühlrippen vorgesehen werden, die in den gerätefesten Ringkanal ragen und dort durch Kühlluft gekühlt werden. Dadurch ist eine gezielte Abführung der Wärme von den Komponenten auf dem Drehkranz nach außen möglich, ohne daß der Innenraum des Gerätes unzulässig erwärmt wird.

Eine zweckmäßige konstruktive Lösung ergibt sich, wenn die Kühlrippen an der Außenseite einer Kammer im Drehkranz angeordnet sind, die von einem Kühlmittel, z. B. von Kühlöl, durchströmt ist, das durch das Gehäuse des Röntgenstrahlers und/oder durch eine Kühlkammer für den Strahlendetektor und/oder der Meßelektronik strömt. Im Ringkanal kann dabei zur kontaktlosen Signalübertragung vom Strahlendetektor ein rotierender Signalübertrager angeordnet sein, der von einander gegenüberliegenden Hochfrequenzantennen am Drehkranz und im Ringkanal gebildet ist.

Die Erfindung ist nachfolgend anhand der Zeichnung näher erläutert. Es zeigen :

Fig. 1 einen Computertomographen nach der Erfindung, und

Fig. 2 einen Schnitt nach der Linie II-II in Figur 1.

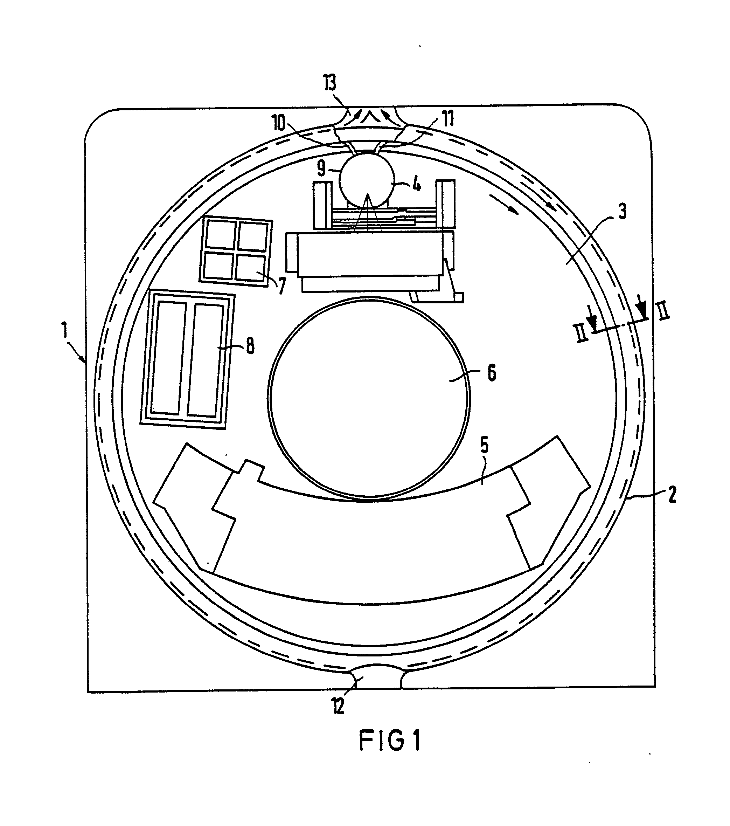

In der Figur 1 ist ein Computertomograph 1 dargestellt, der einen feststehenden Geräteteil 2 und auf einem Drehkranz 3 einen Röntgenstrahler 4 und einen Strahlendetektor mit Meßelektronik 5 aufweist. Zur Abtastung eines in einer Öffnung 6 liegenden Untersuchungsobjektes wird der Drehkranz 3 mit dem Röntgenstrahler 4 und dem Strahlendetektor 5 um das Aufnahmeobjekt gedreht, so daß dieses aus einer Vielzahl von Richtungen durchstrahlt wird. Ein Rechner berechnet aus den jeweils empfangenen Strahlungsintensitäten die Schwächungswerte vorbestimmter Punkte in einer Schicht des Untersuchungsobjektes, die als Computertomogramm bildlich wiedergegeben werden können. Auf dem Drehkranz sind noch eine Spannungsversorgung 7 und eine Zusatzelektronik 8 angeordnet.

Zur Kühlung der Komponenten auf dem Drehkranz 3, insbesondere des Röntgenstrahlers 4, wird durch das Gehäuse 9 des Röntgenstrahlers 4 Öl gepumpt, das durch eine Eintrittsleitung 10 ein- und durch eine Austrittsleitung 11 austritt. Das Kühlöl strömt durch eine im Bereich der Außenseite des Drehkranzes 3 liegende Leitung um diesen herum von der Ölaustrittsleitung 11 zur Öleintrittsleitung 10. Hierzu ist auf dem Drehkranz 3 eine Umwälzpumpe angeordnet.

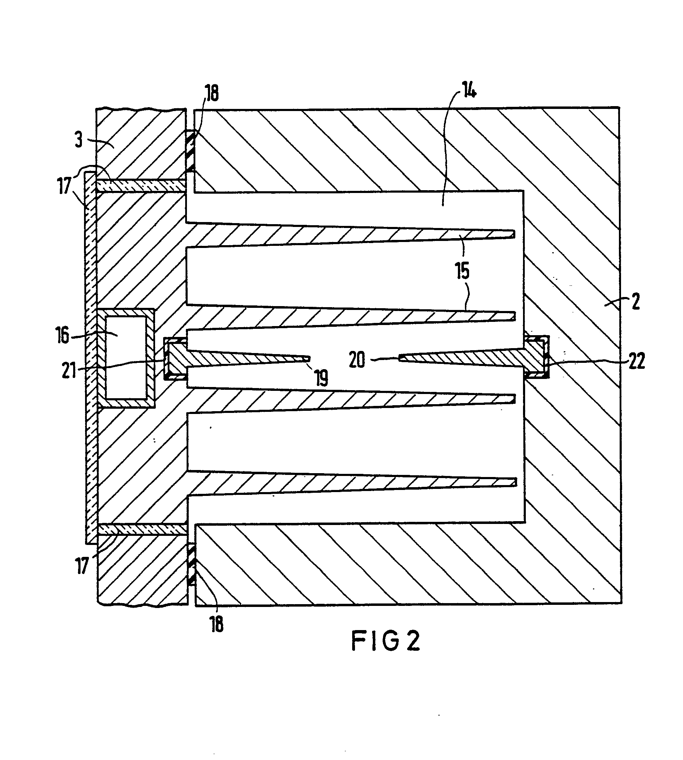

Die Kühlung des Kühlöles erfolgt durch Kühlluft, die durch einen Lufteintrittsstutzen 12 ein- und durch einen Luftaustrittsstutzen 13 austritt. Die Kühlung ist anhand der Figur 2 noch näher erläutert.

Aus der Figur 2 geht hervor, daß der feststehende Geräteteil 2 einen U-förmigen Ringkanal 14 aufweist, durch den die Kühlluft von einem Ventilator von dem Lufteintrittsstutzen 12 zum Luftaustrittsstutzen 13 befördert wird. In den Ringkanal 14 ragen Kühlrippen 15, die am Drehkranz 3 angeordnet sind, und zwar im Bereich der Ölleitung 16. Wenn der Drehkranz 3 rotiert, bewegen sich die Kühlrippen 15 im Ringkanal 14 und werden von der Kühlluft überströmt, wodurch die Wärme vom Kühlöl gezielt abgeführt wird. Eine Wärmeisolierung 17 sorgt dabei dafür, daß die Wärme des Kühlöles sich im wesentlichen nur auf die Kühlrippen 15, nicht jedoch auf die übrigen Teile des Drehkranzes 3 fortpflanzt. Ferner ist zwischen dem feststehenden Teil 2 und dem Drehkranz 3 eine Dichtung 18 vorgesehen, die ein Ausströmen der Kühlluft aus dem Kanal 14 verhindert.

In ähnlicher Weise wie der Röntgenstrahler 4 kann auch der Strahlendetektor und/oder die Meßelektronik 5 durch Kühlöl gekühlt werden.

Im Ringkanal 14 ist ein rotierender Übertrager angeordnet, der von einander gegenüberliegenden Hochfrequenzantennen 19 am Drehkranz 3 und 20 am feststehenden Teil 2 gebildet ist. Die Hochfrequenzantennen 19, 20 sind durch Isolierstücke 21, 22 gegenüber den Komponenten 2, 3 isoliert.

## Patentansprüche

1. Computertomograph mit einem feststehenden Geräteteil (2), in dem ein Drehkranz (3) drehbar gelagert ist, der zu beiden Seiten einer den Patienten aufnehmenden Öffnung (6) einen Röntgenstrahler (4) und einen Strahlendetektor (5) trägt, dadurch gekennzeichnet, daß die dem Drehkranz (3) zugewandte Fläche des feststehenden Geräteteiles (2) einen U-förmigen Querschnitt aufweist und mit der dem feststehenden Geräteteil zugewandten Fläche des Drehkranzes (3) einen Ringkanal (14) bildet, der mindestens eine Einströmöffnung (12) und eine Ausströmöffnung (13) für ein Kühlmedium aufweist und in den am Drehkranz angeordnete Kühlrippen (15) ragen.

2. Computertomograph nach Anspruch 1, dadurch gekennzeichnet, daß die Kühlrippen (15) an der Außenseite einer Kammer (16) im Drehkranz (3) angeordnet sind, die von einem Kühlmittel durchströmt ist, das durch das Gehäuse (9) des Röntgenstrahlers (4) und/oder des Strahlendetektors und/oder der Meßelektronik (5) strömt.

3. Computertomograph nach Anspruch 1 oder 2, dadurch gekennzeichnet, daß im Ringkanal (14) ein rotierender Signalübertrager (19, 20) angeordnet ist.

4. Computertomograph nach Anspruch 3, dadurch gekennzeichnet, daß der Signalübertrager von einander gegenüberliegenden Hochfrequenzantennen (19, 20) am Drehkranz (3) und im Ringkanal (14) gebildet ist.

## Claims

1. Computer tomography equipment having a stationary apparatus part (2) in which there is rotatably mounted a rotary mount (3) which, on both sides of an opening (6) which receives the patient, supports an X-ray tube (4) and a radiation detector (6), characterised in that the stationary apparatus part (2) forms with the rotary mount (3) an annular channel (14) which has at least one inlet opening (12) and one outlet opening (13) for a cooling medium and into which project cooling fins (15) arranged on the rotary mount.

2. Computer tomography equipment according to claim 1, characterised in that the cooling fins (15) are arranged on the outside of a chamber (16) in the rotary mount (3), through which chamber there flows a coolant which flows through the housing (9) of the X-ray tube (4) and/or of the radiation detector and/or of the (sic) measuring electronics (5).

3. Computer tomography equipment according to claim 1 or 2, characterised in that a rotary signal transmitter (19, 20) is arranged in the annular channel (14).

4. Computer tomography equipment according to claim 3, characterised in that the signal transmitter is formed by high-frequency antennae (19, 20), lying opposite each other, on the rotary ring (3) and in the annular channel (14).

## Revendications

1. Tomodensitomètre comportant une partie fixe (2), dans laquelle est montée de façon à pouvoir tourner une couronne rotative (3) qui porte, des deux côtés d'une ouverture (6) de réception du patient, un émetteur radiologique (4) et un détecteur de rayonnement (5), caractérisé par le fait que la partie fixe (2) de l'appareil forme, avec la couronne rotative (3), un canal annulaire (14), qui comporte au moins une ouverture d'entrée (12) et une ouverture de sortie (13) pour un fluide de refroidissement, et dans lequel pénètrent des nervures de refroidissement (15) installées sur la couronne rotative.

2. Tomodensitomètre suivant la revendication 1, caractérisé par le fait que les nervures de refroidissement (15) sont disposées sur la face extérieure d'une chambre (16) située dans la couronne rotative (3) et traversée par un fluide de refroidissement qui traverse le boîtier (9) de l'émetteur radiologique (4) et/ou du détecteur de rayonnement et/ou du système électronique de mesure (5).

3. Tomodensitomètre suivant la revendication 1 ou 2, caractérisé par le fait qu'un dispositif rotatif (19, 20) de transmission de signaux est disposé dans le canal annulaire (14).

4. Tomodensitomètre suivant la revendication 3, caractérisé par le fait que le dispositif de transmission de signaux est formé par des antennes à haute fréquence (19, 20) situées en vis-à-vis l'une de l'autre, installées sur la couronne rotative (3) et dans le canal annulaire (14).

FIG 1

FIG 2